## Europäisches Patentamt
## European Patent Office
## Office européen des brevets

(19)

(11) Veröffentlichungsnummer **0 003 322**
**B1**

(12) **EUROPÄISCHE PATENTSCHRIFT**

(45) Veröffentlichungstag der Patentschrift:
20.05.81

(51) Int. Cl.³ **A 61 M 16/00**

(21) Anmeldenummer: 79100143.1

(22) Anmeldetag: 18.01.79

(54) **Trachealtubus mit Überdruckventil.**

(30) Priorität **25.01.78 DE 2803094**

(43) Veröffentlichungstag der Anmeldung:
**08.08.79 Patentblatt 79/16**

(45) Bekanntmachung des Hinweises auf die Patenterteilung:
**20.05.81 Patentblatt 81/20**

(84) Benannte Vertragsstaaten:
**DE FR GB**

(56) Entgegenhaltungen:
**DE-A-2 353 153**
**DE-A-2 659 440**
**GB-A-733 890**
**US-A-3 794 043**
**US-A-3 985 141**
**US-A-3 989 571**
**US-A-4 064 882**

(73) Patentinhaber: **HOECHST AKTIENGESELLSCHAFT,
Zentrale Patentabteilung Postfach 80 03 20,
D-6230 Frankfurt/Main 80 (DE)**

(72) Erfinder: **Jaeger, Friedrich, Dr., Marxstrasse 7,
D-6370 Oberursel/Taunus (DE)**
Erfinder: **Lammers, Ludwig, Dr., Saalburgweg 3,
D-6270 Idstein/Taunus (DE)**

BUNDESDRUCKEREI BERLIN

Trachealtubus mit Überdruckventil

Die Erfindung betrifft einen Trachealtubus mit aufblasbarer Niederdruckmanschette, bei der der Innendruck mittels eines in die Aufblasleitung eingesetzten Überdruckventils begrenzt wird.

Bei manchen Erkrankungen der Lunge und der Luftwege ist es erforderlich, dem Patienten durch einen Trachealtubus Luft zuzuführen. Der Trachealtubus weist dabei an seinem distalen Ende eine aufblasbare Manschette auf, mit der der Zwischenraum zwischen Innenwand der Luftröhre und Außenwand des Beatmungsrohres abgedichtet wird.

Ältere Trachealtuben haben hierbei eine Manschette, die dadurch hergestellt wird, daß ein Schlauchstück eines elastischen Materials über den Tubusschlauch gezogen und an beiden Enden verklebt wird. Wird eine solche Manschette aufgeblasen, so dehnt sich ihre Oberfläche sofort und bewirkt bei anwachsender Größe der Manschette einen steigenden Luftdruck in ihrem Inneren. Bis zur Anlage der Manschette an der Trachealwand des Patienten hat sich ein bestimmter Innendruck aufgebaut, der oft beträchtlich ist und einige 100 mm Hg betragen kann. Der Anlagedruck der Manschette an der Trachealwand ist zudem ungleichmäßig, da die Manschette infolge ihrer Eigenspannung einen kreisrunden Querschnitt annehmen will, die Querschnittsform der Trachea aber nicht rund ist und somit durch die Manschette bis zur Abdichtung zwangsweise in eine angenähert runde Form gedrückt wird. Der Anlagedruck der Manschette an der Trachealwand ist hierbei nicht meßbar.

Neuere Trachealtuben verwenden eine Niederdruckmanschette. Es handelt sich hierbei um eine ballonförmig vorgeformte Manschette, die beim Einführen des Tubus lose gefaltet am Tubusschlauch anliegt. Unter einer Niederdruckmanschette wird im folgenden eine Manschette verstanden, deren Umfang im ungespannten Zustand im allgemeinen größer, jedenfalls nicht kleiner ist als der Umfang der Trachea des Patienten, für den sie vorgesehen ist. Wird eine vorgeformte Niederdruckmanschette, deren Enden einen kleineren Durchmesser haben als ihre Mitte, mit diesen beiden Enden auf den Tubusschlauch geklebt, zuerst luftleer gemacht und dann langsam aufgeblasen, so entfaltet sich zuerst, praktisch ohne Überdruck, die gefaltet liegende Manschette, bis sie gerade nicht mehr schlapp ist. In diesem Zustand hat sie einen bestimmten Umfang, der als »Umfang in ungespanntem Zustand« bezeichnet werden soll. Bringt man einen solchen Tubus vor dem Aufblasen in ein Glasrohr, dessen Durchmesser kleiner ist als der der ungespannten Niederdruckmanschette, so sieht man, daß die Manschette bei praktisch drucklosem Aufpumpen in ihrem Mittelteil zur Anlage im Glasrohr kommt, wobei je nach den Maßen mehr oder weniger stark ausgebildete, im wesentlichen längs des Glasrohres verlaufende Falten in der Manschettenhaut auftreten. Erhöht man den Druck in der Manschette, so überträgt er sich voll auf die im ursprünglichen Anlageteil ungespannte Haut und von dieser in gleicher Größe auf die Glaswand der Röhre. Mißt man nun den Luftdruck, so hat man gleichzeitig den Wert für den Druck, den die Manschettenhaut auf die Wand der Glasröhre ausübt. Überträgt man den Vorgang auf die Trachealwand und die Niederdruckmanschette, so läßt sich erkennen, daß auch hier der Aufblasdruck der Manschette identisch mit dem auf die Trachealwand ausgeübten Druck von Seiten der Manschette ist.

Dieser Zusammenhang kann nun ausgenutzt werden, um Trachealtuben mit überwachtem Druck zu applizieren. Verschiedene technische Vorrichtungen sind bereits vorgeschlagen worden, die alle das Ziel haben, den Druck mit 15 bis 30 mm Hg so niedrig zu halten, daß keine wesentlichen Beeinträchtigungen der Durchblutung der Trachealwand erfolgen.

So ist aus der US-Patentschrift 3 985 141 ein Trachealtubus mit einem Überdruckventil bekannt, das ein Entweichen überschüssigen Druckes aus dem Trachealballon erlaubt. Auch in der US-Patentschrift 3 794 043 wird ein Ventil für einen Trachealtubus beschrieben, das als Überdruckventil arbeiten kann. Weiterhin ist aus der britischen Patentschrift 733 890 ein Ventil für einen Trachealtubus bekannt, das aus einem Hohlkörper mit seitlicher Öffnung besteht, welche durch ein elastisches schlauchförmiges Element überspannt ist. Schließlich wird auch in der deutschen Offenlegungsschrift 2 353 153 ein Trachealtubus beansprucht, bei dem die Manschette sich bei einem bestimmten Luftüberdruck vom Beatmungsrohr teilweise radial abheben und dadurch das Sicherheitsventil wirken soll.

Trotz dieser vielfältigen Lösungsvorschläge ist ein einfaches und trotzdem völlig zuverlässig arbeitendes Überdruckventil für einen Trachealtubus, das nur zu einem einmaligen Gebrauch bestimmt ist und deshalb preiswert sein muß, bisher nicht entwickelt worden. Die besondere technische Schwierigkeit besteht darin, mit einfachen Mitteln sicherzustellen, daß die gewünschte Ventilwirkung zuverlässig eintritt, wenn der Innendruck der Manschette einen Wert von etwa 25 mm Quecksilber überschreitet. Aufgabe der vorliegenden Erfindung war es deshalb, ein billiges und einfaches, aber zuverlässig arbeitendes Überdruckventil für einen Trachealtubus zu schaffen, der die vorstehend genannten Nachteile vermeidet.

Gegenstand der Erfindung ist ein Trachealtubus zum einmaligen Gebrauch, der an seinem in die Luftröhre ragenden Ende mit einer aufblasbaren Niederdruckmanschette versehen ist und einen zum Aufblasen der Niederdruckmanschette geeigneten Hilfsschlauch mit Rückschlagventil und Überdruckventil aufweist, wobei das Überdruckventil aus einem Hohlkörper mit seitlicher Öffnung besteht, über welche eine elastische, schlauchförmige Haut gespannt ist und zwischen Hohlkörper und elastische Haut ein Dicht- und Trennmittel, vorzugsweise Silikonöl, gebracht ist.

Der genannte Hohlkörper kann vorzugsweise zylindrisch oder ballig-rund sein. Zweckmäßigerweise wird er aus thermoplastischem Kunststoff hergestellt, wobei sich besonders Hart-PVC bewährt hat. Die die Öffnung des Hohlkörpers überspannende elastische schlauchförmige Haut wird vorzugsweise aus Naturgummi hergestellt. Die Herstellung der Haut aus Naturgummi kann z. B. im Tauchverfahren erfolgen.

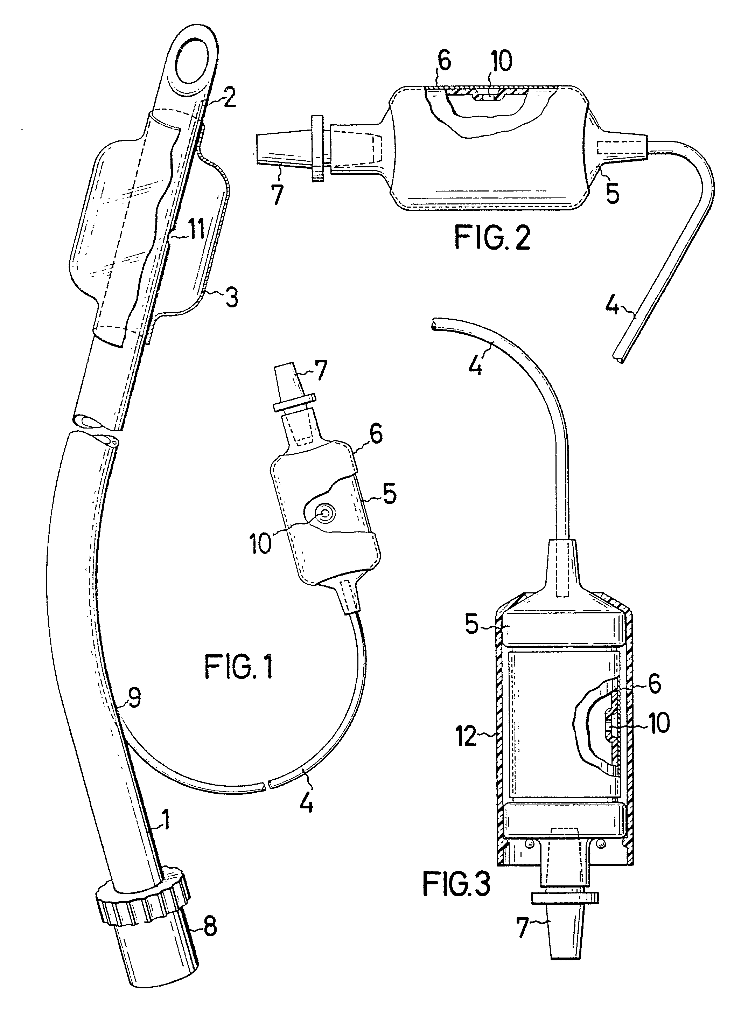

Eine vorzugsweise Ausführungsform des Trachealtubus mit dem Überdruckventil ist in Fig. 1 bis 3 dargestellt.

Das Tubusrohr (1) hat ein großes Lumen zum Durchgang der Atemluft und ein in die Wand eingearbeitetes Hilfslumen zum Aufblasen der Manschette. Am proximalen Ende ist der Konnektor (8) zum Anschluß an die Beatmungsmaschine angebracht. Das Hilfslumen ist an der Spitze (2) geschlossen. Die Spitze selbst ist vorzugsweise röntgen-opak ausgerüstet. Die Manschette (3) ist nahe dem distalen Ende aufgebracht und durch eine Einkerbung (11) im Tubusrohr (1) mit dem Hilfslumen verbunden. An der Stelle (9) wird das Hilfslumen angeschnitten und der Hilfsschlauch (4) so eingeschoben und eingeklebt, daß er mit dem Hilfslumen zum distalen Ende hin verbunden ist, während das Hilfslumen an der Stelle (9) zum proximalen Ende hin abgeschlossen ist.

Der Hilfsschlauch (4) ist an seinem proximalen Ende mit dem Hohlkörper (5) verbunden, vorzugsweise, indem er in diesen eingeführt und eingeklebt wird. Zwischen den Hilfsschlauch und den Hohlkörper kann auch noch ein in den Zeichnungen nicht dargestellter Prüfballon zwischengeschaltet werden, wobei dann der Hilfsschlauch auf der einen Seite in den Prüfballon eingesteckt und verklebt oder verschweißt und der Prüfballon mit der anderen Seite auf den Stutzen des Kohlkörpers gezogen werden kann.

Der Hohlkörper (5) weist eine seitliche Öffnung (10) auf, durch welche Luft aus dem System entweichen kann. Um den Hohlkörper (5) ist eine elastische Haut (6) gespannt, die die Öffnung (10) abdichtet und sich bei einem bestimmten Luftdruck im Hohlkörper und damit kommunizierend in der Manschette abhebt und die Luft entweichen läßt. Dadurch wirkt er als Überdruckventil. Da der Druck der Manschette auf die Trachea den Wert von 25 mm Hg nicht überschreiten soll, darf auch der Auftragsdruck der elastischen Haut auf dem Hohlkörper nicht über 25 mg Hg liegen, vorzugsweise sollte der Auflagedruck 18 bis 20 mm Hg betragen. Der Hohlkörper wird durch das Rückschlagventil (7) abgeschlossen, welches beispielsweise eingeklebt sein kann. Dieses Ventil (7) kann ein normales Rückschlagventil sein oder aber eines, welches durch Einstecken der Spitze einer medizinischen Spritze mechanisch aufgedrückt wird und sich beim Herausziehen der Spritze selbsttätig schließt.

Die elastische Haut (6) kann aus einem Schlauchstück bestehen oder in bestimmter Kontur hergestellt sein, z. B. als Tauchteil aus Latex oder aus einem anderen elastischen Material.

Um zu erreichen, daß die elastische Haut auf dem Hohlkörper einerseits nicht klebt, andererseits aber trotzdem hinreichend gasdicht ist, wird ein dünner Film eines Dicht- und Trennmittels wie Silikonöl zwischen Hohlkörper und Haut gebracht. Zum Schutz der elastischen Haut kann über den Stützkörper noch eine Schutzhülse (12) gezogen werden, beispielsweise durch klemmendes Aufstecken.

Der erfindungsgemäße Trachealtubus mit Niederdruckmanschette verhindert dank der sinnvollen Konstruktion des Überdruckventils, daß der Druck der Manschette auf der Trachea einen Wert von 25 mm Hg überschreitet, stellt aber gleichzeitig eine zuverlässige Abdichtung des Zwischenraumes zwischen Beatmungsrohr und Trachea sicher

## Patentanspruch

Trachealtubus zum einmaligen Gebrauch, der an seinem in die Luftröhre ragenden Ende (2) mit einer aufblasbaren Niederdruckmanschette (3) versehen ist und einen zum Aufblasen der Niederdruckmanschette geeigneten Hilfsschlauch (4) mit Rückschlagventil (7) und Überdruckventil aufweist, dadurch gekennzeichnet, daß das Überdruckventil aus einem Hohlkörper (5) mit seitlicher Öffnung (10) besteht, über welche eine elastische, schlauchförmige Haut (6) gespannt ist, wobei zwischen Hohlkörper (5) und elastische Haut (6) ein Dicht- und Trennmittel, vorzugsweise Silikonöl, gebracht ist.

## Claim

An endotracheal tube for one use which is provided, at its end (2) projecting into the trachea, with an inflatable low-pressure sleeve (3) and has an auxiliary tube (4) which is suitable for inflating the low-pressure sleeve and carries a non-return valve (7) and a relief valve, characterized in that the relief valve consist of a hollow body (5) having a lateral opening (10) around which an elastic tubular skin (6) being stretched and wherein between hollow body (5) and elastic skin (6) a sealing and release agent, preferably silicone oil, is introduced.

## Revendication

Canule trachéale à usage unique qui est munie d'une manchette à basse pression gonflable à son extrémité (2) engagée dans la trachée-artère et qui comporte un tuyau auxiliaire (4) approprié pour le gonflage de la manchette à basse pression et muni d'un clapet anti-retour (7) et d'une soupape de supression, caractérisée en ce que la soupape de surpression est constituée par un corps creux (5) muni d'une ouverture latérale (10) sur laquelle est tendue une peau élastique tubulaire (6), un agent d'étanchéité et de séparation, de préférence une huile de silicone, étant disposé entre le corps creux (5) et la peau élastique (6).

FIG. 1

FIG. 2

FIG. 3